# EUROPEAN PATENT SPECIFICATION

(11) **EP 0 523 184 B1**
(45) Date of publication and mention of the grant of the patent: **30.12.1998**
(21) Application number: 91908528.2
(22) Date of filing: 14.03.1991
(51) Int. Cl.: B01F 17/30, C01F 1/00

(54) **OIL SPEIL RECOVERY METHOD**
VERFAHREN ZUR BESEITIGUNG VON AUSGELAUFENDEM ÖL
PROCEDE POUR LA RECUPERATION DE PETROLE REPANDU

(30) Priority: 05.04.1990 US 505126
(43) Date of publication of application: 20.01.1993
(73) Proprietor: NURTURE, Inc., Missoula, Montana 59802 (US)
(72) Inventor: POTTER, Richard, C., Seeley Lake, MT 59868 (US)
(74) Representative: Whitaker, Iain Mark
(86) International application number: PCT/US91/01744
(87) International publication number: WO 91/15117

(56) References cited:
- EP-A- 0 146 174
- FR-A- 2 287 214
- FR-A- 2 571 253
- US-A- 1 787 585
- US-A- 1 890 860
- US-A- 1 920 926
- US-A- 2 355 029
- US-A- 2 436 818
- US-A- 2 754 215
- US-A- 2 876 164
- US-A- 3 885 052
- US-A- 4 238 509
- US-A- 4 677 065
- PATENT ABSTRACTS OF JAPAN vol. 50, no. 62 25 April 1981 & JP-A-56 015 203 ( EARTH CHEM CORP LTD ) 14 February 1981
- DATABASE WPIL Section Ch, Week 8224, 10 May 1982 Derwent Publications Ltd., London, GB; Class D03, AN 82-49234E & JP-A-57 074 384 (NIPPON KAGAKU SHIRY AND ASAHI ELECTROCHEM IND KK)
- Food Chemistry, 2nd Edition (FENNEMA) 1985, Marcel Dekkar, Inc., pages 298-303.

## Description

### Field of the Invention

The present-invention relates to a method for the clean-up and/or control of oil spills into the environment.

### Background to the Invention

Commercially available oil dispersants are all liquids. These include Corexit ® 9527 (Exxon), a surfactant-solvent product for oil dispersal; Naxchem ® Dispersant K (formerly known as Conco Dispersant K and now sold by Ruetgers-Nease), a product consisting primarily of surfactants and alcohols; Atlant'ol ® AT-7 (Aspra, Inc), a water-based product; Omni-Clean ® OSD (Delta Omega Technologies), a water-based product containing synthetic surfactants and fatty acid soaps; Corexit 9550 (Exxon); and Corexit 7664 (Exxon), a product formerly marketed as an open-sea dispersant but now sold mostly as a beach cleaner.

There is a need for an alternative to these existing dispersants that overcomes some or all of the known drawbacks of these products.

### Summary of the Invention

According to the present invention there is provided a method for dispersing an oil spill in an environmental body of water comprising the steps of:
providing a dispersant comprising a proteinaceous particulate material comprising milled seed material having oil sorptive and emulsifying properties; and applying said dispersant to the oil spill on top of the water to disperse at least a portion of said oil spill.
Preferably the dispersant is derived from a cereal grain by milling and removal of lipids therefrom.
Suitably the seeds are selected from the group consisting of legumes and grains.
More particularly the seeds are suitably selected from the group consisting of canola, beans, oats, rape seed, and soya and most preferably are oats.
Preferably the proteinaceous material is derived from grinding a starting protein source and extracting lipids from the resulting ground material with a lipophilic solvent.
Usefully, the proteinaceous material is buoyant and floats freely on the oil on top of the water. It suitably has a particle size of between about 1 and about 600 µm and suitably a protein content of from 1 to 100%.
In the context of the present invention the oil is generally crude oil or a liquid derivative thereof.
Oil in water emulsions comprising proteinaceous emulsifiers of milled seed material origin are known of in the art of formulating sheet-like topically/applied cosmetic and pharmaceutical preparations. In US 4, 777,046 and US 2, 436,818 the emulsifier is mixed with an oil or wax lipophilic phase before addition of limited amounts of water and subsequent drying. The proteinaceous emulsifiers of the prior art have not however, been used as dispersants for slicks of oil on water.

### Brief Description of the Figures

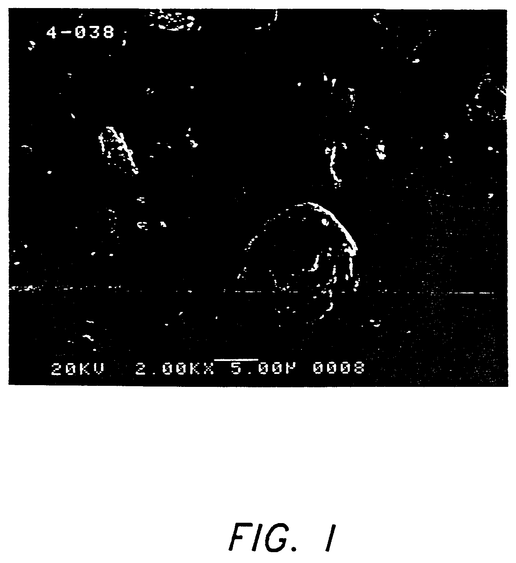

Figure 1 is a photomicrograph of a preferred embodiment of the proteinaceous emulsifier of the present invention which comprises oat seed protein.

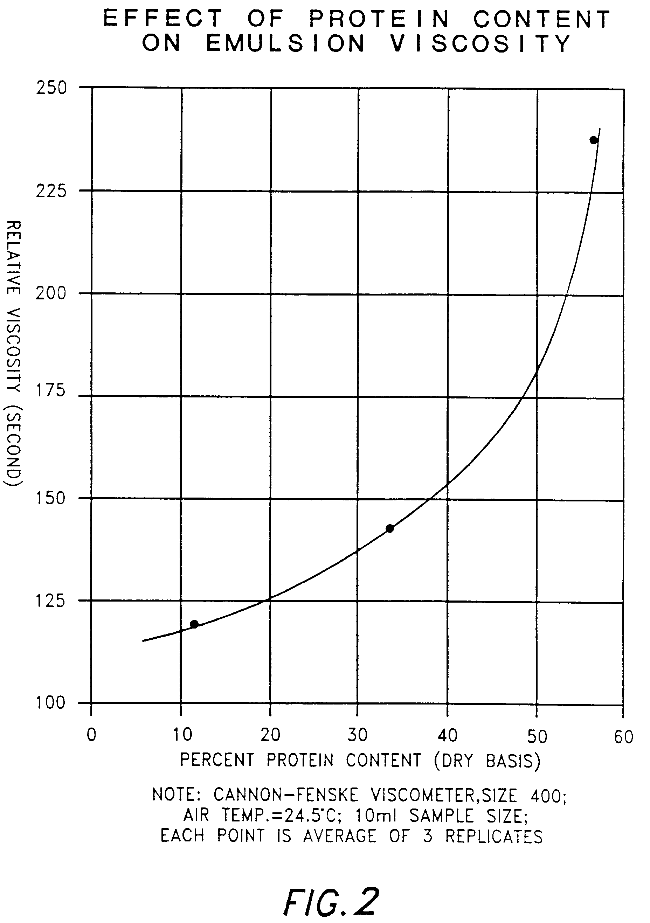

Figure 2 is a graphic representation of the effect of the relative protein content of the proteinaceous emulsifier on the Viscosity of the emulsion of the present invention.

### Description of the Preferred Embodiment

The milled seed protein for use in the method of oil dispersal of the present invention may vary according to considerations of availability and expense associated with the protein as well as the nature of impurities in and other components of the protein source. Preferred proteins include those derived from vegetable or grain sources, particularly from grains or legumes including wheat, canola, beans, oats, peas, rapeseed, and soya, with particularly preferred proteins including oats, peas and beans. Sources of proteins which may be subject to treatment often contain various impurities which may negatively affect emulsion formation. It is desirable, therefore, that where proteins useful with the invention are naturally associated with insoluble components, these components be removable prior to processing.

A number of known processes exist for the preparation of a suitable proteinaceous material for use in the present invention. For example, in U.S. Patent No. 4,089,848 to Bell, the isolation of a proteinaceous fraction from oats is disclosed by extracting lipids from the comminuted oats with a lipophilic solvent, carrying out alkaline and acid precipitation on the residue, and finally isolating the acid soluble protein. Oughton, in U.S. Patent No. 4,154,728, describes another process for separating fractions of differing compositions from comminuted proteinaceous material from a variety of food sources including wheat, rye, barley, triticale, peas and buckwheat. The Oughton process comprises mixing the proteinaceous material with an aliphatic hydrocarbon or alcohol suitable to dissolve the lipids in the material. The wet slurry is distributed by means of centrifugation into fractions which differ primarily in protein composition. A similar process is applied to comminuted oats in U.S. Patent Nos, 4,211,695 and 4,211,801 to Oughton.

To facilitate recovery of the protein in particulate form from the slurry produced in accordance with the foregoing processes, U.S. Patent Nos. 4,208,259 and 4,208,260 to Oughton disclose the application of an electric field to the mixture and collection of a comminuted oat fraction which clings to the anode. An improved method of recovery is disclosed in U.S. Patent No. 4,407,841 to Boocock, comprising the addition of aqueous ethanol to the slurry to agglomerate the proteinaceous material and facilitate separation thereof.

The protein particles are separated to a desired particle size or range of sizes, depending upon the desired properties of the emulsion film.

When the protein particulate is derived from natural grains and legumes, the particles will be irregular in shape, due to crushing and fragmenting during the milling process. However, median particle size can be determined by milling parameters or by using a series of graduated sieves or particle size analysis. Additionally, because of their natural origin, the protein particles of the present invention are fully biodegradable, there are no harmful polymer degradation products that could be released.

Many suitable protein concentrates or protein particulates are commercially available. For example, soya protein concentrate is available in 92% pure form from Protein Technologies International, St. Louis, Missouri, USA. Pea protein concentrates are available from Woodstone Foods, Winnipeg, Canada.

The protein particulate is advantageously dried prior to use to remove water and other indigenous volatiles. In addition, depending upon the protein separation process, residual solvent could reside in the interstices of the particulate which could react adversely with the active ingredients of the emulsion.

Drying can be accomplished by any of a number of known methods, such as oven drying at elevated temperatures or subjecting the powder to a vacuum with or without the addition of heat.

In carrying out the method of the present invention the proteinaceous material is introduced to an area of oil release upon a body of water. Natural or artificial movement of the material into and throughout the oil and water sorbs the oil, thereby forming an emulsifiable concentrate which can then serve to emulsify the oil and produce a fine emulsion, more easily assimilated into the environment.

These emulsions can be dispersed into a large excess of water, in which case a colloidal suspension is formed wherein the water becomes turbid and the emulsion particles remain suspended for long periods of time. This method of providing a colloidal dispersion of oil may be particularly efficacious, in that microbial biodegradation of the oil can more easily occur. It has been observed that a form of the proteinaceous material which has buoyant properties may be particularly efficacious in such applications.

For example, 500 ml of Pacific Ocean seawater was placed in a beaker with a magnetic stir bar. A 1 gram piece of foamed protein particulate derived from oat grain (freeze dried) was added to the beaker after a few ml of 10W-30 motor oil had been dropped on the surface of the water. The protein particulate immediately upon addition to the water sorbed the majority of the oil. Agitation was initiated and continued for ca. 5 minutes. The protein particulate began to break up, with many exposed surfaces appearing white as a result of emulsification. In addition, there were observed many small white particles of the protein particulate admixed throughout the seawater when agitated, giving a flocculated appearance.

After another ca. 5 minutes of additional agitation, the seawater appeared quite turbid as a result of continued emulsification. After an additional ca. 5 minutes of agitation, the effects of the addition of the protein particulate were more pronounced: the water showed increased turbidity; there was an increase of flocculated protein particulate; and very little of the original motor oil appeared to be free in the seawater.

## Claims

1. A method for dispersing an oil spill in an environmental body of water comprising the steps of:
providing a dispersant comprising a proteinaceous particulate material comprising milled seed material having oil sorptive and emulsifying properties; and applying said dispersant to the oil spill on top of the water to disperse at least a portion of said oil spill.

2. The method of claim 1, wherein said dispersant is derived from a cereal grain by milling and removal of lipids therefrom.

3. The method of claim 1, wherein said seeds are selected from the group consisting of legumes and grains.

4. The method of claim 1, 2 or 3 wherein said seeds are selected from the group consisting of canola, beans, oats, rape seed, and soya.

5. The method of claim 4, wherein said seeds are oats.

6. The method of claim 1, 2, 3 or 4 wherein said proteinaceous material is derived from grinding a starting protein source and extracting lipids from the resulting ground material with a lipophilic solvent.

7. The method of any of claims 1 to 6 wherein the particle size of the particulate proteinaceous material is from about 1 to about 600 µm

8. The method of any preceding claim wherein the protein content of the proteinaceous material is from 1 to 100% weight, dry basis

9. The method of any of claims 1 to 8 wherein said proteinaceous material is buoyant and floats freely on the oil on top of water.

10. The method of any preceding claim wherein the oil is crude oil or a liquid derivative thereof.

## Patentansprüche

1. Verfahren zum Dispergieren von ausgelaufenem Öl in umgebenden Gewässern mit den folgenden Schritten:
Vorsehung eines Dispersionsmittels, das eiweißartiges, aus Partikeln bestehendes Material enthält, das gemahlenes Samenmaterial mit ölaufsaugenden und emulgierenden Eigenschaften enthält, und Aufbringen dieses Dispersionsmittels auf das ausgelaufene Öl auf der Oberfläche des Wassers, um wenigstens einen Bereich des ausgelaufenen Öls zu disperieren.

2. Verfahren nach Anspruch 1, wobei das Dispersionsmittel aus einem Getreidekorn durch Mahlen und Entfernen von Lipiden daraus abgeleitet ist.

3. Verfahren nach Anspruch 1, bei dem der Samen aus der Gruppe ausgewählt wird, die aus Gemüse und Getreide besteht.

4. Verfahren nach Anspruch 1, 2 oder 3, bei dem der Samen aus der Gruppe ausgewählt wird, die aus Canola, Bohnen, Hafer, Rapssamenkörnern und Soja besteht.

5. Verfahren nach Anspruch 4, wobei als Samen Hafer verwendet wird.

6. Verfahren nach Anspruch 1, 2, 3 oder 4, bei dem das eiweißartige Material durch Zerfasern einer Ausgangsproteinquelle und Entziehen von Lipiden aus dem sich ergebenden zerfaserten Material mit einer lipophilen Lösung abgeleitet wird.

7. Verfahren nach einem der Ansprüche 1 bis 6, bei dem die Partikelgröße des aus Partikeln bestehenden eiweißartigen Materials von etwa 1 bis 600 µm reicht.

8. Verfahren nach einem der vorhergehenden Ansprüche, bei dem der Proteingehalt des eiweißartigen Materials von 1 bis 100 Gew.-% trockene Basis ist.

9. Verfahren nach einem der Ansprüche 1 bis 8, bei dem das eiweißartige Material schwimmfähig ist und frei auf dem Öl auf der Wasseroberfläche schwimmt.

10. Verfahren nach einem der vorhergehenden Ansprüche, wobei das Öl Rohöl oder ein flüssiges Derivat davon ist.

## Revendications

1. Procédé pour disperser une coulée de pétrole répandu dans une masse d'eau de l'environnement, comprenant les étapes consistant à:
fournir un dispersant comprenant une matière particulaire protéinique comprenant une matière de graines broyées ayant des propriétés de sorption et émulsionnantes du pétrole; et appliquer ledit dispersant à la coulée de pétrole, sur la surface de l'eau pour disperser au moins une partie de la coulée de pétrole.

2. Procédé selon la revendication 1, dans lequel ledit dispersant est issu de grains de céréales par broyage et par élimination des lipides de ceux-ci.

3. Procédé selon la revendication 1, dans lequel lesdites graines sont choisies parmi le groupe comprenant les légumes et les céréales.

4. Procédé selon la revendication 1, 2 ou 3 dans lequel lesdites graines sont choisies parmi le groupe comprenant le colza canola, les haricots, l'avoine, les graines de colza et le soja.

5. Procédé selon la revendication 4, dans lequel lesdites graines sont de l'avoine.

6. Procédé selon la revendication 1, 2, 3 ou 4, dans lequel ladite matière protéinique est obtenue en broyant une source de protéines de base et en extrayant des lipides de la matière broyée résultante avec un solvant lipophile.

7. Procédé selon l'une quelconque des revendications 1 à 6, dans lequel la taille des particules de la matière particulaire protéinique est comprise entre environ 1 et environ 600 µm.

8. Procédé selon l'une quelconque des revendications précédentes, dans lequel la teneur en protéine de la matière protéinique est comprise entre 1 et 100% en poids, à sec.

9. Procédé selon l'une quelconque des revendications 1 à 8, dans lequel ladite matière protéinique est flottante et flotte librement sur le pétrole à la surface de l'eau.

10. Procédé selon l'une quelconque des revendications précédentes, dans lequel le pétrole est du pétrole brut ou un liquide dérivé de celui-ci.
